# EUROPÄISCHE PATENTSCHRIFT

(11) **EP 1 512 432 B1**
(45) Veröffentlichungstag und Bekanntmachung des Hinweises auf die Patenterteilung: **06.09.2006**
(21) Anmeldenummer: 04450133.6
(22) Anmeldetag: 29.06.2004
(51) Int. Cl.: A61N 1/44

(54) **Einrichtung zum Behandeln eines Patienten mit elektrischem Strom**
Apparatus for treating a patient with electric current
Appareil de traitement d'un patient à l'aide d'un courant électrique

(30) Priorität: 03.09.2003 AT 13862003
(43) Veröffentlichungstag der Anmeldung: 09.03.2005
(73) Patentinhaber: Luttermann, Joachim, 8082 Kirchbach (AT)
(72) Erfinder: Luttermann, Joachim, 8082 Kirchbach (AT)
(74) Vertreter: Gibler, Ferdinand

(56) Entgegenhaltungen:
- EP-A- 0 269 306
- CH-A- 101 511
- DE-A- 3 043 300
- FR-A- 926 956
- US-A- 5 741 317

## Beschreibung

Die Erfindung betrifft eine Einrichtung zur Behandlung eines Patienten mit elektrischem Strom, insbesondere Reizstrom, welche Einrichtung ein Gefäß zur Aufnahme eines elektrisch leitenden Fluids aufweist, in das über mit einer Stromquelle freiliegende verbundene Elektroden Strom einleitbar ist.

Eine derartige Einrichtung wurde z.B. durch die DE 30 43 300 C bekannt. Bei dieser bekannten Einrichtung ist eine Wanne vorgesehen in der unter der üblichen Füllhöhe der Wanne Stromschienen angeordnet sind, an denen Elektroden einhängbar sind. Dabei sind Stromstärken im Bereich von 1.5 Ampere vorgesehen.

Bei dieser Einrichtung stellt der zu behandelnde Patient praktisch einen Widerstand in der durch das elektrisch leitende Fluid, z.B. Wasser mit wählbaren Zusätzen, gebildeten Widerstandsstrecke zwischen an unterschiedlichen Potenzialen angeschlossenen Elektroden dar. Damit stellt der Patient im Wesentlichen einen Parallelwiderstand dar, der in einem Widerstand, dem elektrisch leitenden Fluid eingebettet ist. Da der Widerstand des Fluids von sehr vielen Faktoren, u.a. auch von der Füllhöhe der Wanne, abhängt, ist die tatsächliche Beaufschlagung des Patienten mit Strom, kaum mit ausreichender Genauigkeit erfassbar, abgesehen davon, dass es bei der bekannten Lösung nicht ausgeschlossen werden kann, dass der Patient die Elektroden berührt, wodurch eine sehr erhebliche Änderung seiner Strombelastung eintritt.

Ziel der Erfindung ist es, diese Nachteile zu vermeiden und eine Einrichtung der eingangs erwähnten Art vorzuschlagen, mit der eine weitgehend genau einstellbare Beaufschlagung des Patienten mit Strom möglich ist.

Erfindungsgemäß wird dies dadurch erreicht, dass das Gefäß mittels einer elektrisch isolierenden Trennwand in zwei Abteilungen unterteilt ist und das Fluid in jeder dieser Abteilungen eine Elektrode zugeordnet ist und die beiden Elektroden mit einer gemeinsamen regelbaren Stromquelle elektrisch verbunden sind.

Durch die vorgeschlagenen Maßnahmen ist sichergestellt, dass als Widerstand im Wesentlichen der zu behandelnde Patient zwischen den Elektroden geschaltet ist, wodurch ein relativ genaues Einstellen der zur Anwendung kommenden Ströme auf einfache Weise erreicht werden kann.

Insbesondere kann vorgesehen sein, dass die Stromquelle ein zweiphasiges Rechtecksignal mit einer Taktfrequenz von ca. 4Hz liefert, wobei die Taktung vorzugsweise 1 : 1 beträgt. Dabei entspricht die Frequenz von ∼4Hz der halben Erdfrequenz, wodurch besonders gute physiologische und therapeutische Effekte erzielt werden. Die pathogene Wirksamkeit von Pilzen, (Mykosen) Parasiten, Bakterien und Viren im Körper wird herabgesetzt und stärkt somit das Immunsystem.

In besonderer Ausgestaltung der Erfindung kann vorgesehen sein, dass die an den beiden Ausgängen der Stromquelle abgreifbaren Signale im Wesentlichen symmetrisch verlaufen. Eine symmetrische Signalerzeugung lässt sich schaltungstechnisch besonders einfach realisieren.

In Weiterführung der Erfindung kann vorgesehen sein, dass unter den Böden der beiden Abteilungen des Gefäßes je ein Elektrodenraum vorgesehen ist, in dem je eine Elektrode, vorzugsweise in Form eines Drahtes, gehalten ist, wobei der Elektrodenraum über Bohrungen mit der zugeordneten Abteilung des Gefäßes in Verbindung steht. Dadurch wird auf einfache Weise eine Berührung der Elektroden durch den Patienten sicher vermieden und es ergibt sich eine sehr einfache Konstruktion. Dabei sind auch die Elektroden gegen eine Beschädigung geschützt.

Vorteilhafter Weise kann weiters vorgesehen sein, dass für jeden Elektrodenraum wenigstens eine verschließbare Öffnung vorgesehen ist, die für die Aufnahme bzw. den Austausch von Elektroden und/oder zur Reinigung des Elektrodenraums geeignet ist. So können hygienische Probleme sicher vermieden werden und Wartungsarbeiten mit nur geringem Aufwand durchgeführt werden.

In Weiterführung der Erfindung kann vorgesehen sein, dass die Abteilungen des Gefäßes über mindestens eine elektrisch isolierende Zuleitung mit einer Druckluftquelle in Verbindung stehen

Dadurch ist es möglich, das im Gefäß befindliche Fluid, z.B. mit verschiedenen Zusätzen versetztes Wasser, mit Luft zu versetzen und so für eine entsprechende Bewegung des Fluids zu sorgen. Durch die Steigerung des Leitwerts des Fluids durch geeignete Zusätze, wie insbesondere Salze, wird der Effekt der Einrichtung erhöht. Eine Saug-Druckluftmassage wird die Aufnahmefähigkeit der Hautoberfläche weiter erhöht.

In diesem Zusammenhang ist es zur Erzielung einer im Wesentlichen gleichmäßigen Beaufschlagung des in den beiden Abteilungen des Gefäßes befindlichen Fluids mit Luft zweckmäßigerweise vorgesehen, dass die an die Druckluftquelle angeschlossenen Zuleitungen mit den Elektrodenräumen verbunden sind.

Die Erfindung wird unter Bezugnahme auf die beigeschlossenen Zeichnungen, in welchen besonders bevorzugte Ausführungsbeispiele dargestellt sind, näher beschrieben. Dabei zeigt:
Fig. 1 eine Draufsicht einer erfindungsgemäße Einrichtung,
Fig. 2 eine Seitenansicht der Einrichtung nach der Fig. 1,
Fig. 3 ein Detail der Einrichtung nach den Fig. 1 und 2,
Fig. 4 ein Diagramm der Signale der Stromquelle, und
Fig. 5 eine Seitenansicht einer Elektrode mit einer Zuleitung für Druckluft.

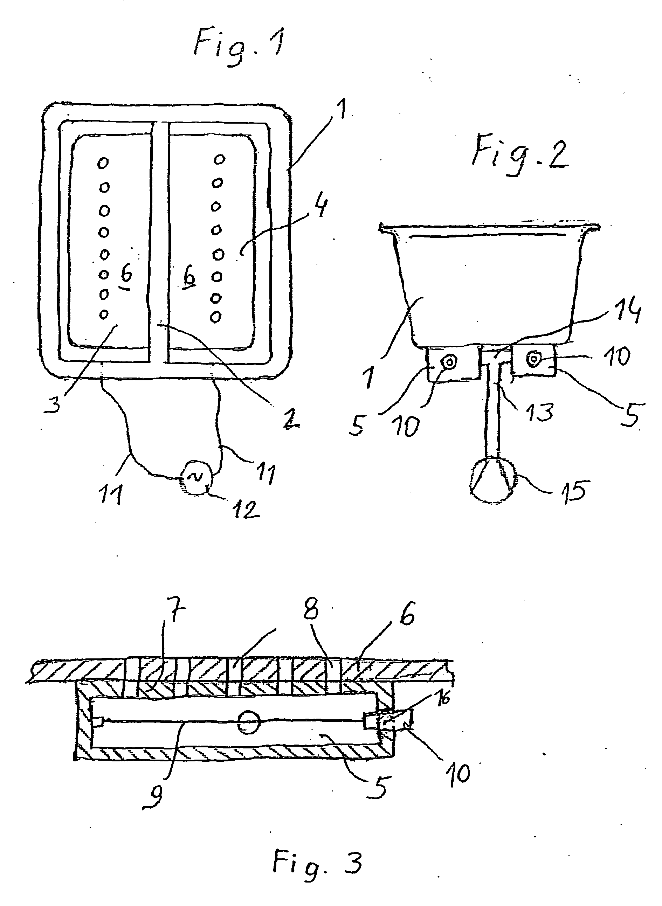

Wie aus der Fig. 1 und 2 zu ersehen ist, umfasst die erfindungsgemäße Einrichtung ein Gefäß 1 das eine sich bis zu seinem oberen Rand erstreckende Zwischenwand 2 aufweist, die das Gefäß 1 in zwei Abteilungen 3, 4 unterteilt.

Unter dem Boden 6 des Gefäßes 1 sind zwei Elektrodenräume 5 angeordnet, wobei der Boden 6 des Gefäßes 1 und der Oberteil 7 des Elektrodenraumes 5 mit Bohrungen 8 versehen sind, sodass die Elektrodenräume 5 mit den zugeordneten Abteilungen 3, 4 in Verbindung stehen und beim Füllen der Abteilungen 3, 4 des Gefäßes 1 auch die entsprechenden Elektrodenräume 5 gefüllt werden.

In den Elektrodenräumen 5 ist je eine Elektrode 9 in Form eines Silberdrahtes freiliegend, der mit einem Stecker 10 in Verbindung steht. Silber eignet sich wegen seiner hervorragenden elektrischen Leitfähigkeit und seiner Korrosionsbeständigkeit besonders gut als Elektrodenmaterial. An diesen ist eine Anschlussleitung 11 anschließbar, die eine Verbindung mit einer regelbaren Stromquelle 12 herstellt.

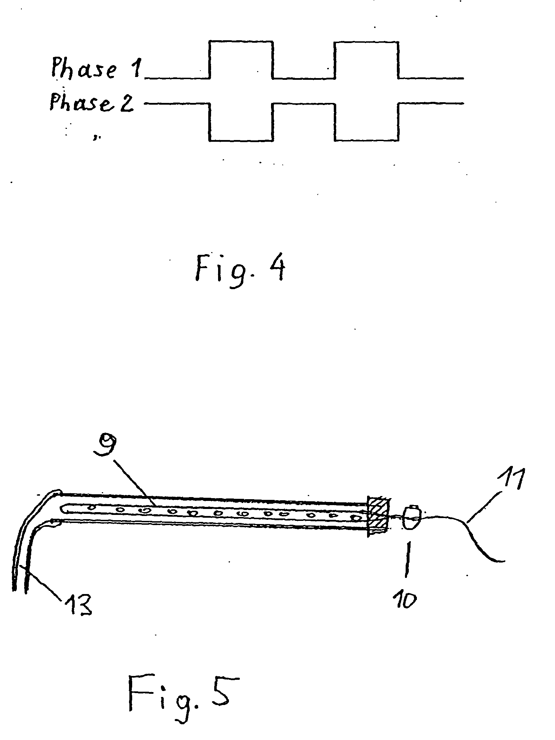

Diese liefert vorzugsweise ein zweiphasiges Rechtecksignal mit einer Frequenz von ca. 4 Hz, dessen beide Phasen an je einer Elektrode 9 in jedem der beiden Elektrodenräume 5 beim Betrieb der Einrichtung anliegen. Dabei sind die beiden Phasen des zweiphasigen Signals der Stromquelle, wie aus der Fig. 4 zu ersehen ist, symmetrisch zueinander, wobei das Tastverhältnis der Rechtecksignale im wesentlichen 1 : 1 beträgt.

Die Elektrodenräume 5 sind an eine gemeinsame Zuleitung 13 über ein T-Stück 14 angeschlossen. Diese Zuleitung 13 ist an einen Druckluftquelle 15 angeschlossen.

Im Betrieb der Einrichtung wird Wasser mit verschiedenen Zusätzen zur Erhöhung seiner elektrischen Leitfähigkeit in die beiden Abteilunge 3, 4 des Gefäßes 1 gefüllt. Nach dem Einschalten der regelbaren Stromquelle 12 kommt es zu einem intermittierenden Stromfluss über den Körper des Patienten, der z.B. mit seinen Füßen in den beiden Abteilungen 3, 4 des Gefäßes steht. Dabei kann die Stromstärke sehr einfach eingestellt werden. Salzlösungen erhöhen den Leitwert des Wassers, sodass der elektrische Strom leichter übertragen und über die Haut beispielsweise an Reflexzonen, Meridianbahnen, Lymphflüssigkeit, Blutbahnen und

Körpergewebe weitergeleitet werden kann.

Gleichzeitig kann Druckluft in die Elektrodenräume 5 eingebracht werden, die über die Bohrungen 8 in die Abteilungen 3, 4 aufsteigt und das Wasser in den Abteilungen verwirbelt.

Zur leichteren Reinigung der Elektrodenräume 5 oder für eine leichte Austauschbarkeit der Elektroden 9 können weiters Öffnungen 16 vorgesehen sein, die beispielsweise durch einen Stecker 10 dicht verschließbar sind. Der Stecker 10, in den die Elektrode 9 dicht vergossen ist, kann beispielsweise in die Öffnung 16 geschraubt sein.

Wie in Fig. 5 dargestellt, können die Elektroden 9 insbesondere in die Zuleitungen 13 für Druckluft integriert sein.

Zur Erhöhung des Komforts des Patienten kann das Gefäß 1 weiters mit einer vorzugsweise regelbaren Heizungseinrichtung versehen sein, die das Fluid auf eine gewünschte Temperatur erwärmt.

## Patentansprüche

1. Einrichtung zur Behandlung eines Patienten mit elektrischem Strom, insbesondere Reizstrom, welche Einrichtung ein Gefäß (1) zur Aufnahme eines elektrisch leitenden Fluids aufweist, in das über mit einer Stromquelle (12) freiliegende verbundene Elektroden (9) Strom einleitbar ist, **dadurch gekennzeichnet, dass** das Gefäß (1) mittels einer elektrisch isolierenden Trennwand (2) in zwei Abteilungen (3, 4) unterteilt ist und das Fluid in jeder dieser Abteilungen (3, 4) eine Elektrode (9) zugeordnet ist und die beiden Elektroden (9) mit einer gemeinsamen regelbaren Stromquelle (12) elektrisch verbunden sind.

2. Einrichtung gemäß Anspruch 1, **dadurch gekennzeichnet, dass** die Stromquelle (12) ein zweiphasiges Rechtecksignal mit einer Taktfrequenz von ca. 4Hz liefert, wobei die Tastung vorzugsweise 1 : 1 beträgt.

3. Einrichtung gemäß Anspruch 2, **dadurch gekennzeichnet, dass** die an den beiden Ausgängen der Stromquelle (12) abgreifbaren Signale im Wesentlichen symmetrisch verlaufen.

4. Einrichtung gemäß einem der Ansprüche 1 bis 3, **dadurch gekennzeichnet, dass** unter den Böden (6) der beiden Abteilungen (3, 4) des Gefäßes (1) je ein Elektrodenraum (5) vorgesehen ist, in dem je eine Elektrode (9), vorzugsweise in Form eines Drahtes, gehalten ist, wobei der Elektrodenraum (5) über Bohrungen (8) mit der zugeordneten Abteilung (3, 4) des Gefäßes (1) in Verbindung steht.

5. Einrichtung gemäß Anspruch 4, **dadurch gekennzeichnet, dass** für jeden Elektrodenraum (5) wenigstens eine verschließbare Öffnung (16) vorgesehen ist, die für die Aufnahme bzw. den Austausch von Elektroden (9) und/oder zur Reinigung des Elektrodenraums (5) geeignet ist.

6. Einrichtung gemäß einem der Ansprüche 1 bis 5, **dadurch gekennzeichnet, dass** die Abteilungen (3, 4) des Gefäßes (1) über mindestens eine elektrisch isolierende Zuleitung (13) mit einer Druckluftquelle (15) in Verbindung stehen.

7. Einrichtung gemäß Anspruch 5, **dadurch gekennzeichnet, dass** die an die Druckluftquelle (15) angeschlossenen Zuleitungen (13) mit den Elektrodenräumen (5) verbunden sind.

## Claims

1. A device for treating a patient with electric current, especially stimulation current, which device comprises a vessel (1) for receiving an electrically conductive fluid, into which current can be introduced by way of bare electrodes (9) connected with a source of current (12), **characterized in that** the vessel (1) is subdivided into two compartments (3, 4) by means of an electrically insulating separating wall (2) and the fluid is associated with an electrode (9) in each of said compartments (3, 4) and the two electrodes (9) are electrically connected with a commonly adjustable source of current (12).

2. A device according to claim 1, **characterized in that** the source of current (12) supplies a two-phase rectangular signal with a clock frequency of approx. 4 Hz, with the keying preferably being 1:1.

3. A device according to claim 2, **characterized in that** the signals which can be tapped at the two outputs of the source of current (12) extend substantially in a symmetrical way.

4. A device according to one of the claims 1 to 3, **characterized in that** an electrode chamber (5) is each provided beneath of the floors (6) of the two compartments (3, 4) of the vessel (1), in which an electrode (9) each, preferably in the form of a wire, is held, with the electrode chamber (5) being in connection via bores (8) with the associated compartment (3, 4) of the vessel (1).

5. A device according to claim 4, **characterized in that** at least one closeable opening (16) is provided for each electrode chamber (5), which opening is suitable for receiving or exchanging electrodes (9) and/or for cleaning the electrode chamber (5).

6. A device according to one of the claims 1 to 5, **characterized in that** the compartments (3, 4) of the vessel (1) are in connection with a compressed air source (15) by way of at least one electrically insulating feed line (13).

7. A device according to claim 5, **characterized in that** the feed lines (13) connected to the compressed air source (15) is connected with the electrode chambers (5).

## Revendications

1. Dispositif de traitement d'un patient par application de courants électriques, notamment par stimulation électrique, du type comportant un récipient (1) destiné à recevoir un fluide conducteur d'électricité dans lequel peut être introduit un courant par l'intermédiaire d'électrodes (9) exposées reliées à une source de courant (12), **caractérisé en ce que** le récipient (1) est divisé en deux compartiments (3, 4) moyennant une cloison (2) électriquement isolante et que le fluide dans chacun des compartiments (3, 4) est associé à une électrode (9) et que les deux électrodes (9) sont reliées électriquement à une source de courant électrique (12) commune réglable.

2. Dispositif selon la revendication 1, **caractérisé en ce que** la source de courant (12) fournit un signal rectangulaire biphase cadencé à une fréquence de 4 Hz environ, la cadence étant de préférence de 1:1.

3. Dispositif selon la revendication 2, **caractérisé en ce que** les signaux susceptibles d'être prélevés aux deux sorties de la source de courant (12) sont sensiblement symétriques.

4. Dispositif selon l'une quelconque des revendications 1 à 3, **caractérisé en ce qu'**une enceinte (5) pour électrodes est prévue en dessous du plancher (6) de chacun des deux compartiments (3, 4) du récipient (I), chaque enceinte (5) contenant une électrode (9), de préférence sous forme de fil, et communiquant par des trous (8) avec le compartiment (3, 4) associé du récipient (1).

5. Dispositif selon la revendication 4, **caractérisé en ce qu'**au moins une ouverture (16) obturable et apte à la réception ou au remplacement des électrodes (9) et/ou au nettoyage de l'enceinte (5) est prévue pour chaque enceinte (5) pour électrodes.

6. Dispositif selon l'une quelconque des revendications 1 à 5, **caractérisé en ce que** les compartiments (3, 4) du récipient (1) sont reliés à une source d'air comprimé (15) par l'intermédiaire d'au moins une conduite d'amenée (13) électriquement isolante.

7. Dispositif selon la revendication 5, **caractérisé en ce que** les conduites d'amenée (13) raccordées à la source d'air comprimé (15) sont reliées aux enceintes (5) pour électrodes.
